# EUROPEAN PATENT APPLICATION

(11) **EP 3 412 198 A1**
(43) Date of publication of application: **12.12.2018**
(21) Application number: 18174823.7
(22) Date of filing: 29.05.2018
(51) Int. Cl.: A61B 3/00, A61B 3/06

(54) **VISUAL FUNCTION TEST APPARATUS**

(30) Priority: 02.06.2017 JP 2017110230
(71) Applicant: Nidek Co., Ltd., Gamagori-shi, Aichi, 443-0038 (JP)
(72) Inventor: AKITA, Jyunichi, Gamagori-shi, Aichi 443-0038 (JP); IWATA, Shinya, Gamagori-shi, Aichi 443-0038 (JP)
(74) Representative: Prüfer & Partner mbB Patentanwälte · Rechtsanwälte

(57) **Abstract**

A visual function test apparatus comprises: a light projecting optical system (70) configured to irradiate stimulation light and background light to an examination area of a fundus (Er) of an eye (E) of an examinee; reaction obtaining means (7c) configured to obtain a reaction of the examinee to the stimulation light; and control means (80) configured to control at least an irradiation position of the stimulation light to be irradiated from the light projecting optical system to the examination area. The light projecting optical system (70) is configured to irradiate the background light at a luminance corresponding to dark adaptation and irradiate the stimulation light and the background light in a first wavelength band having a lower limit on a longer wavelength side than a sensitivity peak of blue cone cells and an upper limit on a shorter wavelength side than a sensitivity peak of green cone cells, the first wavelength band being a wavelength band including at least a sensitivity peak of rod cells.

## Description

### TECHNICAL FIELD

The present disclosure relates to a visual function test apparatus configured to apply a light stimulus to an examinee's eye to examine the visual function of the examinee's eye.

### BACKGROUND

Heretofore, there has been known a visual function test apparatus configured to project stimulation light to a fundus of an eye of an examinee (an examinee's eye) and obtain a reaction of the examinee to the stimulation light to thereby examine the visual function of the examinee.

Some studies have revealed that, in a case where visual functions deteriorate due to retinal disorder including age-related retinal pigmentary dystrophy and AMD (age-related macular degeneration), the rod cells contributing to dark adaptation of a retina tend to sharply degrease in sensitivity as compared with the cone cells contributing to light adaptation of the retina.

From such a background, a visual function test under dark adaptation has been proposed. For example, Non-patent Document 1 discloses an attempt to place a short pass filter that transmits the light having a wavelength of 530 nm or less but shields the light having a wavelength longer than 530 nm in an optical path of the stimulation light to selectively stimulate the rod cells.

### Related Art Documents

### Non-Patent Documents

Non-Patent Document 1: Michael D Crossland et. al. "Retinal specific measurement of dark-adapted visual function: validation of a modified microperimeter" BMC Opthalmology, 2011, 11:5

### SUMMARY

However, in the short pass filter employed in the apparatus disclosed in Non-patent Document 1, the stimulation light having passed through the short pass filter may stimulate not only the rod cells but also blue cone cells. In such a case, the function of the rod cells could not be evaluated independently.

The present disclosure has been made to address the above problems and has a purpose to provide a visual function test apparatus capable of appropriately performing a visual function test under a dark adaptation condition.

To achieve the above purpose, a visual function test apparatus provided as a typical aspect of the present disclosure is configured as below.
(1) The visual function test apparatus comprises: a light projecting optical system configured to irradiate stimulation light and background light to an examination area of a fundus of an eye of an examinee; reaction obtaining means configured to obtain a reaction of the examinee to the stimulation light; and control means configured to control at least an irradiation position of the stimulation light to be irradiated from the light projecting optical system to the examination area, wherein the light projecting optical system is configured to irradiate the background light at a luminance corresponding to dark adaptation and irradiate the stimulation light and the background light in a first wavelength band having a lower limit on a longer wavelength side than a sensitivity peak of blue cone cells and an upper limit on a shorter wavelength side than a sensitivity peak of green cone cells, the first wavelength band being a wavelength band including at least a sensitivity peak of rod cells.
(2) In the apparatus configured in (1), preferably, the light projecting optical system includes a bandpass filter configured to transmit the first wavelength band and further shield light on a longer wavelength side and a shorter wavelength side than the first wavelength band.
(3) In the apparatus configured in (1) or (2), preferably, the light projecting optical system includes a neutral density filter having a property of darkening light in the first wavelength band.
(4) Preferably, the apparatus configured in (1) further includes irradiation condition switching means configured to switch an irradiation condition for the stimulation light and the background light in the light projecting optical system between a first irradiation condition for irradiating the stimulation light and the background light in the first wavelength band to perform a visual function test under a dark adaptation condition and a second irradiation condition for irradiating the stimulation light and the background light in a second wavelength band different from the first wavelength band and to which various blue, green, and red cone cells are sensitive to perform a visual function test under a light adaptation condition.
(5) In the apparatus configured in (4), preferably, the irradiation condition switching means includes at least a bandpass filter configured to transmit the first wavelength band and further shield light on a longer wavelength side and a shorter wavelength side than the first wavelength band and an insertion/removal mechanism for inserting and removing the bandpass filter with respect to an optical path of the light projecting optical system.
(6) In the apparatus configured in (4), preferably, the irradiation condition switching means further includes a neutral density filter having a property of darkening light in the first wavelength band and an insertion/removal mechanism for inserting and removing the neutral density filter with respect to an optical path of the light projecting optical system.
(7) In the apparatus configured in (4), preferably, the irradiation condition switching means includes a filter integrally including a bandpass filter configured to transmit the first wavelength band and further shield light on a longer wavelength side and a shorter wavelength side than the first wavelength band and a neutral density filter having a property of darkening the light in the first wavelength band, and when the filter is inserted or removed with respect to an optical path of the light projecting optical system, the bandpass filter and the neutral density filter are inserted or removed together.
(8) In the apparatus configured in (7), preferably, the neutral density filter and the bandpass filter are formed in different layers from each other.
(9) In the apparatus configured in one of (4) to (8), preferably, the irradiation condition switching means further includes a light source of the stimulation light and the background light, and the control means, and at least the light source is controlled to switch the irradiation condition between the first irradiation condition and the second irradiation condition.
(10) In the apparatus configured in (6) or (7), preferably, the irradiation condition switching means further includes a light source of the stimulation light and the background light, and the control means, and an amount of light to be emitted from the light source is controlled in combination with insertion and removal of the neutral density filter to set the background light to a luminance corresponding to dark adaptation under the first irradiation condition.
(11) Preferably, the apparatus configured in one of (1) to (10) further includes an imaging optical system configured to irradiate infrared light from an illumination light source to the examinee's eye and receive reflection light formed by the infrared light from the examinee's eye to obtain a front image of the examinee's eye, wherein the infrared light to be emitted to the examinee's eye by the imaging optical system is light having a wavelength on a longer wavelength side than a wavelength band to which red cone cells are substantially sensitive.
(12) Preferably, the apparatus configured in one of (3), (5), (7), and (8) further includes optical-path combining means configured to combine an optical path of the imaging optical system and an optical path of the light projecting optical system, wherein the bandpass filter is placed in an independent optical path of the light projecting optical system.
   Another aspect of the present disclosure provides a visual function test method configured as below.
(13) The visual function test method uses a visual function test apparatus comprising: a light projecting optical system configured to irradiate stimulation light and background light to an examination area of a fundus of an eye of an examinee; reaction obtaining means configured to obtain a reaction of the examinee to the stimulation light; and control means configured to control at least an irradiation position of the stimulation light to be irradiated from the light projecting optical system to the examination area, wherein the light projecting optical system is configured to irradiate the background light at a luminance corresponding to dark adaptation and irradiate the stimulation light and the background light in a first wavelength band having a lower limit on a longer wavelength side than a sensitivity peak of blue cone cells and an upper limit on a shorter wavelength side than a sensitivity peak of green cone cells, the first wavelength band being a wavelength band including at least a sensitivity peak of rod cells.
(14) In the method configured in (13), preferably, the light projecting optical system includes a bandpass filter configured to transmit the first wavelength band and further shield light on a longer wavelength side and a shorter wavelength side than the first wavelength band.
(15) In the method configured in (13) or (14), preferably, the light projecting optical system includes a neutral density filter having a property of darkening light in the first wavelength band.
(16) In the method configured in (13), preferably, the visual function test apparatus further includes irradiation condition switching means configured to switch an irradiation condition for the stimulation light and the background light in the light projecting optical system between a first irradiation condition for irradiating the stimulation light and the background light in the first wavelength band to perform a visual function test under a dark adaptation condition and a second irradiation condition for irradiating the stimulation light and the background light in a second wavelength band different from the first wavelength band and to which various blue, green, and red cone cells are sensitive to perform a visual function test under a light adaptation condition.
(17) In the method configured in (16), preferably, the irradiation condition switching means includes at least a bandpass filter configured to transmit the first wavelength band and further shield light on a longer wavelength side and a shorter wavelength side than the first wavelength band and an insertion/removal mechanism for inserting and removing the bandpass filter with respect to an optical path of the light projecting optical system.
(18) In the method configured in (16), preferably, the irradiation condition switching means further includes a neutral density filter having a property of darkening light in the first wavelength band and an insertion/removal mechanism for inserting and removing the neutral density filter with respect to an optical path of the light projecting optical system.
(19) In the method configured in (16), preferably, the irradiation condition switching means includes a filter integrally including a bandpass filter configured to transmit the first wavelength band and further shield light on a longer wavelength side and a shorter wavelength side than the first wavelength band and a neutral density filter having a property of darkening the light in the first wavelength band, and when the filter is inserted or removed with respect to an optical path of the light projecting optical system, the bandpass filter and the neutral density filter are inserted or removed together.
(20) In the method configured in (19), preferably, the neutral density filter and the bandpass filter are formed in different layers from each other.
(21) In the method configured in one of (16) to (20), preferably, the irradiation condition switching means further includes a light source of the stimulation light and the background light, and the control means, and at least the light source is controlled to switch the irradiation condition between the first irradiation condition and the second irradiation condition.
(22) In the method configured in (18) or (19), preferably, the irradiation condition switching means further includes a light source of the stimulation light and the background light, and the control means, and an amount of light to be emitted from the light source is controlled in combination with insertion and removal of the neutral density filter to set the background light to a luminance corresponding to dark adaptation under the first irradiation condition.
(23) In the foregoing method configured in one of (13) to (22), preferably, the visual function test apparatus further includes an imaging optical system configured to irradiate infrared light from an illumination light source to the examinee's eye and receive reflection light formed by the infrared light from the examinee's eye to obtain a front image of the examinee's eye, wherein the infrared light to be emitted to the examinee's eye by the imaging optical system is light having a wavelength on a longer wavelength side than a wavelength band to which red cone cells are substantially sensitive.
(24) In the method configured in one of (15), (17), (19), and (20), preferably, the visual function test apparatus further includes optical-path combining means configured to combine an optical path of the imaging optical system and an optical path of the light projecting optical system, wherein the bandpass filter is placed in an independent optical path of the light projecting optical system.

According to the present disclosure, it is possible to appropriately perform a visual function test under a dark adaptation condition.

### BRIEF DESCRIPTION OF THE DRAWINGS

FIG. 1 is a graph showing wavelength sensitivity characteristics of each of cone cells and rod cells;
FIG. 2 is a diagram showing a distribution of cone cells and a distribution of rod cells in a retina;
FIG. 3 is a diagram showing a display example of isopters;
FIG. 4 is an external configuration diagram of an apparatus in an example;
FIG. 5 is a diagram showing optical systems in the apparatus in the example;
FIG. 6 is a graph showing wavelength transmission characteristics of an ND filter in the example;
FIG. 7 is a graph showing wavelength transmission characteristics of a bandpass filter in the example;
FIG. 8 is a diagram showing a control system in the apparatus in the example;
FIG. 9 is a flowchart to explain operations of the apparatus in the example; and
FIG. 10 is a diagram to explain how to set a testing pattern in a static test.

### DETAILED DESCRIPTION OF THE EXEMPLARY EMBODIMENTS

### [Outline]

The present disclosure will be described based on an embodiment. A visual function test apparatus in this embodiment is configured to project stimulation light (a stimulation target) to an area to be examined (an examination area) of a fundus of an eye of an examinee (an examinee's eye), and obtain a reaction of the examinee to the stimulation light to examine the visual function of the examinee. Hereinafter, the visual function test apparatus in the embodiment is referred to as a "present apparatus". The present apparatus includes at least a light projecting optical system, a reaction obtaining part, and a control unit. The present apparatus may be for example a static perimeter, kinetic perimeter, or another testing device.

The present apparatus is configured to execute at least a visual function test under a dark adaptation condition (hereinafter, referred to as a "Scotopic test"). In the Scotopic test, the function of rod cells contributing to the dark adaptation is examined. As described below, the present apparatus may be configured to further execute a visual function test under a light adaptation condition (hereinafter, referred to as a "Photopic test"). In the Photopic test, the function of cone cells contributing to the light adaptation is examined.

The present apparatus may further include an imaging optical system. An image of the examinee's eye captured by the imaging optical system during a test may also be utilized for example in control of a light projecting optical system, determination of adequacy of a test, and others. The imaging optical system may also be used to photograph a front image of an anterior segment of an eye or photograph a front image of a fundus.

### <Light Projecting Optical System>

The light projecting optical system is configured to irradiate light from a light source (also referred to as a "stimulation light source") as stimulation light (a stimulation target) to an examination area of a fundus of an examinee's eye. The light projecting optical system can change a projection position of the stimulation light to be projected onto the fundus. Further, the shape and the size of the stimulation target may also be changed. The light projecting optical system may irradiate the background light in a visual function test to the entire examination area.

The projection position of the stimulation target on the fundus may be changed by movement of an emission position of the stimulation light in a direction intersecting the optical axis of the light projecting optical system. Further, an optical scanner for deflecting the stimulation light may be placed in an optical path of the light projecting optical system, so that the projection position of the stimulation target on the fundus may also be changed by changing of the orientation of the optical scanner. When a laser light source is adopted as the stimulation light source, the foregoing optical scanner is provided in the light projecting optical system.

In the Scotopic test, the light projecting optical system can irradiate the background light at a luminance corresponding to the dark adaptation and irradiate the stimulation light and the background light in a first wavelength band described below (a first irradiation condition). The first wavelength band is a wavelength band having a lower limit on a longer wavelength side than a sensitivity peak of blue cone cells and an upper limit on a shorter wavelength side than a sensitivity peak of green cone cells, and including at least a sensitivity peak of rod cells. A luminance peak of the stimulation light in the first wavelength band may nearly coincide with a sensitivity peak of the rod cells.

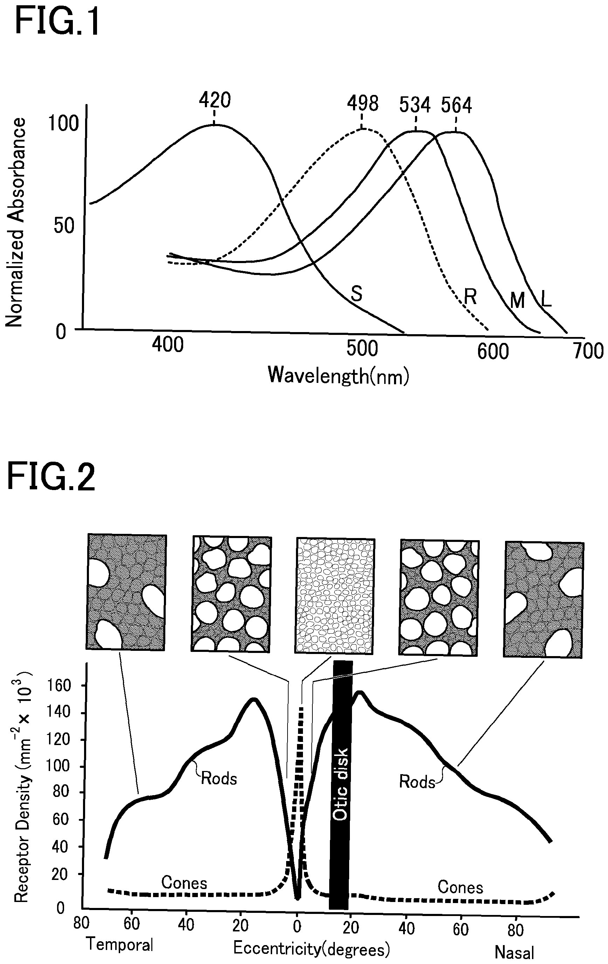

FIG. 1 is a graph showing the sensitivities of various types of cone cells; blue cone cells, green cone cells, and red cone cells, and the sensitivity of rod cells at each wavelength. Three curves plotted with solid lines in FIG. 1 indicate, from a left one in the drawing sheet, the sensitivity of blue cone cells (S), the sensitivity of green cone cells (M), and the sensitivity of red cone cells (L). A curve plotted with a dotted line indicates the sensitivity of rod cells (R). Each of the sensitivities is normalized. The graph in FIG. 1 reveals that it is preferable that the lower limit of the first wavelength band is on a longer wavelength side than 420 nm and the upper limit of the same is on a shorter wavelength side than 534 nm. Herein, the upper limit and the lower limit are defined with reference to the position of a half width in a wavelength intensity distribution of the stimulation light.

More preferably, the lower limit of the first wavelength band is on a longer wavelength side than an intersection of the curve representing the normalized rod cell sensitivity (R) and the curve representing the normalized blue cone cell sensitivity (S). Furthermore, it is preferable that the upper limit of the first wavelength band is on a shorter wavelength side than an intersection of the curve representing the normalized rod cell sensitivity (R) and the curve representing the normalized green cone cell sensitivity (M).

The second wavelength band which is a wavelength band of the stimulation light in the Photopic test is a wavelength band to which various types of blue, green, and red cone cells are sensitive (a second irradiation condition). The wavelength spectrum of light in the second wavelength band may be a continuous distribution such as white light or a discrete distribution with discretely-distributed peaks.

To achieve the stimulation light in the first wavelength band, the light projecting optical system may include a bandpass filter to allow the light having passed through the bandpass filter to form the stimulation light to be irradiated to the examination area. In this case, the light source of the light projecting optical system (the stimulation light source) may be configured to emit broadband light more than the first wavelength band. To change over the wavelength band of the stimulation light to the second wavelength band, an insertion/removal mechanism for inserting/removing the bandpass filter with respect to the optical path of the light projecting optical system may be provided. At that time, when the bandpass filter is inserted into the optical path, the stimulation light in the first wavelength band is produced. In contrast, when the bandpass filter is removed from the optical path, the stimulation light in the second wavelength band is produced.

As an alternative, a light source that emits light in a band region near the sensitive peak (498 nm) of the rod cells may be provided as the light source of the light projecting optical system (the stimulation light source) to achieve the stimulation light in the first wavelength band. As another alternative, a second stimulation light source that emits stimulation light in the second wavelength band may be further provided. In this case, the light sources to be activated to emit light may be switched from one to the other according to a test. Furthermore, insertion/removal of the bandpass filter and switchover of the wavelength bands in the light sources may be combined.

In the Scotopic test, the light projecting optical system can limit the background light to the luminance corresponding to the dark adaptation in order to perform a test under the dark adaptation condition. At that time, a neutral density filter may be placed in the optical path of the light projecting optical system to limit the luminance of the background light or limit the amount of light to be emitted from a light source of background light (as which the light source of stimulation light may double). Both the limitations may be combined. Moreover, the wavelength band including the sensitivity peak (498 nm) of rod cells is also sensed by the cone cells. Accordingly, in the Scotopic test, when the stimulation light in the first wavelength band is projected while the luminance of the stimulation light is high, not only the rod cells but also the cone cells may be stimulated. This may result in an inappropriate result of the function test for the rod cells. Therefore, the light projecting optical system may limit not only the background light but also the luminance of the stimulation light simultaneously through the foregoing structure.

The background luminance (e.g., brightness of the background light) in the Scotopic test is obviously lower than that in the Photopic test. Accordingly, when the Photopic test and the Scotopic test are to be performed by a single apparatus, this apparatus may include a structure for changing the luminance of the background light. Specifically, as one example, the apparatus may include the foregoing neutral density filter and the foregoing insertion/removal mechanism for inserting and removing the neutral density filter. As another example, a light source capable of changing the luminance of light to be emitted may be provided as a light source of the light projecting optical system (more specifically, a light source of the background light). These examples may be combined.

When the Photopic test and the Scotopic test are to be performed by a single apparatus, the stimulation light demanded in the Photopic test and the stimulation light demanded in the Scotopic test are largely different in required luminance. Thus, there is a conceivable case where the single apparatus cannot address both the tests by changing only the luminance in the light source. For instance, a dynamic range of the light source may be insufficient or the output power may be unstable during low emission (herein, mainly, during emission in the Scotopic test). Thus, it is preferable to adjust the luminance of light by combining the neutral density filter and the insertion/removal mechanism thereof with the light source capable of changing the luminance of light to be emitted. Further, when the luminance is to be adjusted by combination of the neutral density filter and the insertion/removal mechanism thereof with the light source capable of changing the luminance of light to be emitted, a production error of the neutral density filter can be corrected in the light source. Specifically, during production or the like, it may be arranged to measure the luminance of light from a light source having passed through the neutral density filter and correspondingly adjust the emission amount of light from the light source in a Scotopic test mode to a desired value.

In a case where the light projecting optical system is provided with both the bandpass filter and the neutral density filter, these two types of filters may be integrated in a single optical member. Since it is difficult to materialize a single component having both two types of properties, the bandpass filter and the neutral density filter are preferably formed as different layers from each other. For example, it may be configured such that the bandpass filter is formed on one surface of a glass material and the neutral density filter is formed on the opposite surface of the same. As an alternative, one of the filters may be a glass material itself. In this case where the bandpass filter and the neutral density filter are formed in a single glass material, the two filters can be inserted/removed all together by a single drive mechanism. Thus, a single apparatus used to perform the Photopic test and the Scotopic test is advantageously simple in structure.

The drive mechanism for inserting and removing various filters (including the bandpass filter and the neutral density filter) placed in the light projecting optical system may be an actuator to be driven based on a signal from the control unit or may be a mechanical mechanism to be directly operated by for example an examiner.

### <Imaging Optical System>

The imaging optical system is configured to irradiate infrared light from an illumination light source to an examinee's eye and receive reflection light of the infrared light reflected from the examinee's eye to obtain a front image of the examinee's eye. The front image of the examinee's eye may be an anterior segment image or a fundus image. It is preferable that a wavelength band of the infrared light to be irradiated from the imaging optical system to the examinee's eye during the Scotopic test is sufficiently far from the sensitivity peak of the red cone cells. Specifically, it is preferably the light having a longer wavelength than the wavelength band to which the red cone cells are substantially sensitive. When the infrared light has a wavelength in the wavelength band of such a degree that an examinee does not perceive redness during the Scotopic test, the function of the rod cells can be more appropriately examined. To be concrete, the wavelength band of the infrared light is preferably in a range of 871 nm or higher.

The present apparatus may include an optical-path combining part and thus the optical path of the imaging optical system and the optical path of the light projecting optical system may be combined by the optical-path combining part. In this case, specifically, the stimulation light from the stimulation light source and the infrared light from the illumination light source are irradiated to the examinee's eye through a common optical path of the light projecting optical system and the imaging optical system. When the bandpass filter is utilized in the optical path of the light projecting optical system, the bandpass filter is placed in an independent optical path of the light projecting optical system. In this case, the bandpass filter does not block or interfere with the infrared light of the imaging optical system. In another case where the bandpass filter is placed in the common optical path of the light projecting optical system and the imaging optical system, the bandpass filter preferably has a wavelength transmission property that transmits not only the first wavelength band but also a wavelength band of such a degree that an examinee does not perceive redness, and shields other wavelength bands.

### <Reaction Obtaining Part>

The reaction obtaining part is configured to obtain a reaction of an examinee to the stimulation light. When a visual function test to be performed by a vision test apparatus is a subjective test, the reaction obtaining part obtains a subjective reaction of the examinee. The reaction obtained by the reaction obtaining part may be a subjective reaction of the examinee who recognizes the irradiation of stimulation light or a physiological reaction caused when the photoreceptor cells are stimulated by the stimulation light. One examples of the reaction obtaining part for obtaining a former reaction may include a UI (a user interface) to be operated by an examinee to respond to the irradiation of stimulation light and a control unit that receives a signal from the UI.

### <Control Unit>

The control unit is configured to control the light projecting optical system and control at least an irradiation position of the stimulation light irradiated from the light projecting optical system to an examination area. For example, the control unit changes the irradiation position of the stimulation light on the fundus by controlling an emission position of the stimulation light in a target unit including a light source or by controlling an optical scanner provided in the optical path of the light projecting optical system.

Further, the control unit may control the irradiation conditions of the stimulation light and the background light. To be specific, for example, the light source of the stimulation light, the light source of the background light, or both these light sources are controlled by the control unit. Control of the light sources may be light-amount control or wavelength switching control of the light emitted from the light source(s). The control unit may also control insertion/removal of the filters such as the bandpass filter and the neutral density filter.

The control unit may control the irradiation condition of the stimulation light and change over the test mode. At least the test mode is switched between two test modes; a Scotopic test mode (a first test mode) and a Photopic test mode (a second test mode). In the Photopic test mode, the control unit irradiates the light in the second wavelength band to an examination area of a fundus. In the Scotopic test mode, the control unit irradiates the light in the first wavelength band to an examination area of a fundus. In the Scotopic test mode, furthermore, the luminance of the stimulation light and the luminance of the background light are set lower than those in the Photopic test mode.

### <Visual Function Test>

A visual field test which is a typical example of the visual function test may be executed by the present apparatus. The visual field test is roughly classified into a static visual field test and a kinetic visual field test. Any of those tests may be executed by the present apparatus. In the static visual field test, the control unit controls the light projecting optical system to project a stimulation target (a spot light or the like) having a fixed size and a changeable intensity (brightness) onto an examination area on a retina. The examinee operates the UI (e.g., a button, etc.) when visually recognizes the stimulation target. This processing is performed sequentially at a plurality of positions on the retina to obtain a retinal luminosity at each area. In the kinetic visual field test, a projection position of the stimulation target on the retina is moved. The examinee operates the UI (e.g., a button, etc.) when visually recognizes the stimulation target. Accordingly, a visually recognizable range (the shape of a visual field) of the examinee's eye is obtained. Further, the control unit may continually obtain a fundus image through the imaging optical system while the visual function test (herein, the visual field test) is performed and track the projection position of the stimulation light on the fundus. This enables accurate stimulation to any portion of the examinee's eye E.

### <Test method taking account of the distribution of photoreceptor cells>

The present apparatus may execute a visual function test by taking account of a density distribution of photoreceptor cells (specifically, a density distribution of cone cells, a density distribution of rod cells, or both the density distributions).

In the static visual field test, for instance, various test parameters may be set based on the density distribution of the photoreceptor cells.

As one example, the control unit may control the light projecting optical system based on the density distribution of the photoreceptor cells to thereby set at least one of the luminance, the size, and the shape of the stimulation targets (one example of the test parameters) according to the density of the photoreceptor cells of the examination area. In this case, the stimulation targets are densely projected in a region where the density of photoreceptor cells is high, while the stimulation targets are sparsely projected in a region where the density of photoreceptor cells is low.

As one example that the test parameters are set based on the density distribution of photoreceptor cells, a projection time of the stimulation target(s) (one example of the test parameters) for each examination area may be set according to the density of photoreceptor cells in each examination area. Furthermore, a threshold value of a response time by an examinee (one example of the test parameters), the threshold value being set in advance to determine whether a response of an examinee is positive, negative, or false-positive, may be set according to the density of photoreceptor cells in each examination area. For example, the threshold value of the response time may be a smaller value for an examination area where the density of photoreceptor cells is higher. This enables the test to be more promptly performed. Since the visual function test is executed as above by taking account of the density distribution of photoreceptor cells, the test can be more efficiently performed.

For example, the density distribution of photoreceptor cells may be a value unrelated to an examinee's eye or may be an actual measured value of an examinee's eye. The value unrelated to an examinee's eye may be an average value of measurement data of a plurality of patients or a literature value. Moreover, in recent years, a fundus photographing apparatus, such as AO-SLO and AO-OCT, for photographing a retina at a cell level has been proposed. An image photographed by such an apparatus may be analyzed to obtain the density distribution of photoreceptor cells in an examinee's eye (an actual measured value).

Meanwhile, the density distribution of cone cells and the density distribution of rod cells are different from each other as shown in FIG. 2. More specifically, the cone cells are concentrated on a central fovea, while the rod cells are distributed less in the central fovea and its vicinity and more in a peripheral area.

In the present apparatus, therefore, it may be arranged such that the test parameters in the Photopic test mode (the second test mode) are set in consideration of the density distribution of the cone cells and the test parameters in the Scotopic test mode (the first test mode) are set in consideration of the density distribution of the rod cells. For example, the Photopic test mode and the Scotopic test mode may also be different in at least the density of cells on which a stimulation target(s) is to be projected per examination area.

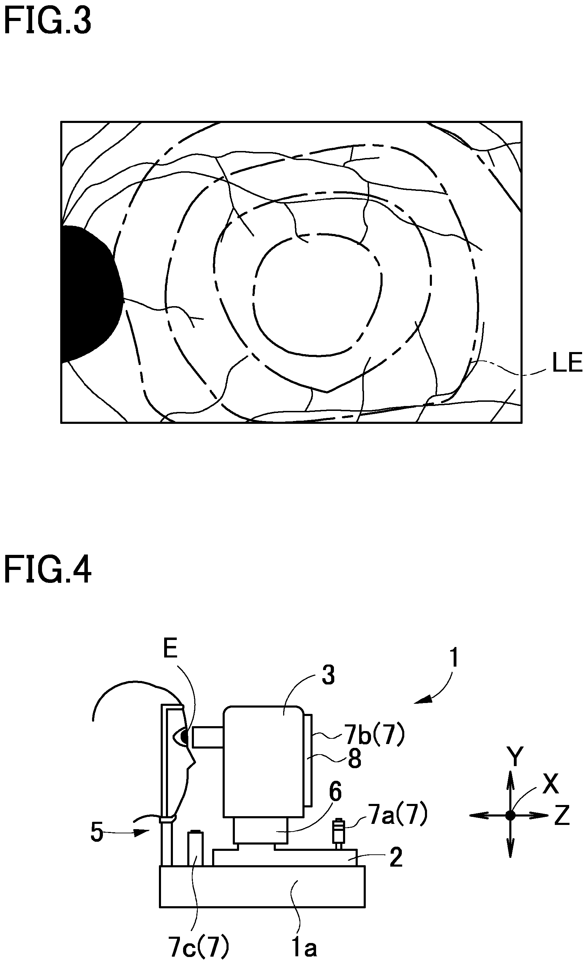

As an example of the Scotopic test taking account of the density distribution of photoreceptor cells (herein, at least the density distribution of rod cells), a kinetic test may be performed by the present apparatus. In this case, the control unit controls the light projecting optical system to dynamically move the stimulation light from the peripheral area to the central area of the fundus (alternatively, from the central area to the peripheral area). Further, the control unit obtains a response position data indicating the position of the stimulation light when the examinee operates the UI (e.g., a button, etc.) during movement of the stimulation light. The control unit changes the luminance of the stimulation light and repeats the test. This processing is performed in each of meridian directions. Based on the response position data obtained in a series of the tests, a luminosity distribution of the rod cells can be determined. The luminosity distribution based on the response position data may be displayed as isopters on a monitor. The isopters may be displayed for example in association with an image of a fundus (see FIG. 3). In FIG. 3, the lines indicated by a reference sign LE are isopters.

As shown in FIG. 2, the distribution range of rod cells is wider than that of cone cells and also the rod cells are distributed less in and around the central fovea and more in the peripheral area. The rod cells are highly responsive to a moving object. Thus, when the Scotopic test is performed by the kinetic test, the function of the rod cells can be promptly and appropriately evaluated.

When both the kinetic test and the static test are performed as the Scotopic test, the test parameters for the static test may be set based on a result of the kinetic test performed in advance. For example, a range of a retina to be exposed to the stimulation light in the static test, an initial value of the luminance of the stimulation light, and an initial size or shape of the stimulation light, and others may be set based on the result of the kinetic test.

### <Display of Test Results>

Data indicating results of the visual function test by the present apparatus may be displayed, on a monitor, together with a fundus image and so that a test result in each examination area is association with the fundus image. For instance, when the kinetic test is performed in the Scotopic test mode, the isopters indicating the results of the Scotopic test using the kinetic test may be merged with the fundus image and displayed together on the monitor. The fundus image may be a front image of the fundus photographed by the imaging optical system or another image. For instance, data indicating results of the visual function test may be displayed in association with three-dimensional data on the fundus obtained by the OCT (optical coherence tomography).

### <Conclusions>

In the vision test apparatus in the present embodiment, the light in the first wavelength band (that is, a wavelength band defined on a longer wavelength side than the sensitivity peak of the blue cone cells and on a shorter wavelength side than the sensitivity peak of the green cone cells, and the wavelength band includes at least the sensitivity peak of rod cells) is irradiated to an examination area as the stimulation light in the Scotopic test. Accordingly, only the rod cells can be selectively stimulated, so that the function of rod cells can be appropriately examined. Consequently, for example, the Scotopic test enables early detection of a retinal disease of an examinee. Further, follow-up tests enable more appropriate ascertainment of the progression of the disease.

### [Examples]

Next, a visual function test apparatus 1 (hereinafter, simply referred to as an "apparatus 1") which is one example of the present disclosure will be described with reference to the accompanying drawings. The apparatus 1 is an apparatus called a micro-perimeter and can perform by itself fundus observation, fundus photographing, and a visual field test.

### <Configuration of Outer Appearance >

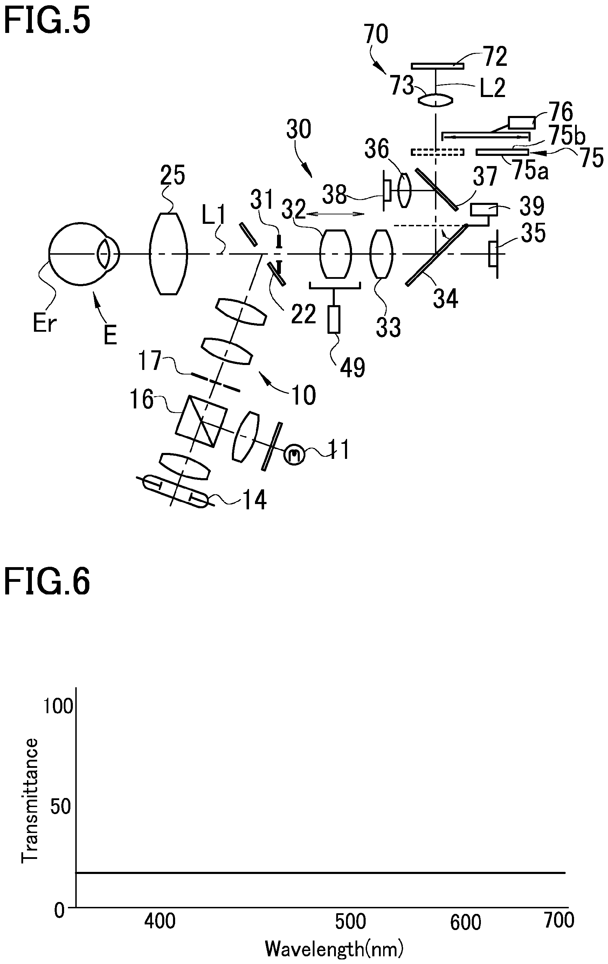

Referring to FIG. 4, the configuration of the outer appearance of the apparatus will be described below. This apparatus 1 mainly includes at least an examination unit 3 and an operation unit 7 (corresponding to the UI in the example). The examination unit 3 is internally provided with main optical systems of the apparatus 1 (see FIG. 5). The details of the structure of the examination unit 3 will be described later.

In the present example, the apparatus 1 includes a base table 1a, a movable table 2, a face support unit 5, and a drive unit 6. They are utilized to adjust the position of the examination unit 3 to the examinee's eye E. On the base table 1a, the examination unit 3 is mounted through the movable table 2. The movable table 2 is provided to be movable in a right-left direction (an X direction) and back and a front-back direction (a Z direction, a working distance direction) relative to the base table 1a. The movable table 2 is moved to adjust the positional relationship between an eye E of an examinee and the examination unit 3 in the X direction and the Z direction. Further, the drive unit 6 is provided on the movable table 2. The drive unit 6 is activated to move the examination unit 3 in the right-left direction (the X direction), an up-down direction (a Y direction), and the front-back direction (the Z direction) relative to the examinee's eye E. The face support unit 5 is fixed to the base table 1a to support the face of the examinee.

The apparatus 1 includes, as the UI, a joystick 7a, a controller 7b, and a response button 7c. The joystick 7a and the controller 7b are operated by an examiner and the response button 7c is operated by the examinee. The joystick 7a is used to relatively move the examination unit 3 relative to the examinee's eye E. When the joystick 7a is tilted, the movable table 2 is caused to slide in the X and Z directions on the base table 1a by a sliding mechanism not shown. The joystick 7a is provided with a rotary knob placed on a peripheral surface and a switch located at a top. When the rotary knob is rotated, the drive unit 6 is activated to move the examination unit 3 in the Y direction. When the switch is operated, an operation of photographing a fundus image or other operations are performed.

The controller 7b is operated to set various photographing conditions and test conditions and others. The controller 7b in the present embodiment is a touch panel incorporated in the monitor 8. Another UI, such as a mouse and a keyboard, may be utilized together with the touch panel or instead thereof. The monitor 8 (display means) displays an observation image of the examinee's eye E, a photographed image of the same, various test results, and others. The response button 7c is utilized for a patient or examinee to enter a response signal in the visual function test and others. That is, this button 7c is used as a part of "reaction obtaining means" in the example.

In the Scotopic test, a housing of the apparatus 1 may be colored in black in order to suppress stimulation to the cone cells. The monitor 8 (the display means) is controlled so that the screen appears at a low luminance at least during the Scotopic test to reduce irradiation of the light to the examinee's eye E. The apparatus 1 may also have an additional outer appearance configuration appropriate to the Scotopic test.

### <Optical Systems>

Next, a schematic structure of the optical systems in the apparatus 1 will be described below with reference to FIG. 5.

The apparatus 1 includes an illumination optical system 10, an observation-photographing optical system 30, and an examination optical system 70. In the apparatus 1, the examination optical system 70 is also used in a fixation optical system for projecting a fixation target to the examinee's eye E.

In the apparatus 1, the illumination optical system 10 and the observation-photographing optical system 30 are mainly used to photograph a fundus image of the examinee's eye E. The illumination optical system 10 illuminates the fundus Er of the examinee's eye E. The observation-photographing optical system 30 photographs the fundus Er illuminated by the illumination optical system 10.

### <Illumination Optical System>

The illumination optical system 10 in the present example includes an illumination light source 11 that irradiates infrared light as illumination light to be used for observation. The illumination optical system 10 further includes a photographing light source 14 that irradiates visible light (e.g., white light) as illumination light to be used for photographing. The illumination optical system 10 further includes a dichroic mirror 16, a ring slit 17, a perforated mirror 22, and an objective lens 25. The dichroic mirror 16 allows the infrared light emitted from the illumination light source 11 and the visible light emitted from the photographing light source 14 to travel along the same optical path (i.e., from the dichroic mirror 16 to the objective lens 25) and fall on the fundus Er of the examinee's eye E.

In the present embodiment, the infrared light emitted from the illumination light source 11 is the light in a wavelength band of such a degree that an examinee does not perceive redness. Concretely, the light in a wavelength band of 871 nm or higher is emitted. Accordingly, even when observation using the infrared light is performed during the Scotopic test, the cone cells are not stimulated by the observation light, so that the function of rod cells can be properly examined.

### <Observation-Photographing Optical System>

The observation-photographing optical system 30 (an "imaging optical system" in the example) includes a first light receiving element (an imaging device 38) that receives fundus reflection light formed by the infrared light for observation and a second light receiving element (an imaging device 35) that receives fundus reflection light formed by the visible light for photographing. In the present example, the first light receiving element (the imaging device 38) and the second light receiving element (the imaging device 35) are separate devices. As an alternative, a single light receiving element may be adopted to receive both the infrared light and the visible light. The first light receiving element (the imaging device 38) and the second light receiving element (the imaging device 35) are placed one on each of two optical paths branched by an optical path branching part 34. The imaging device 38 and the imaging device 35 are also placed in respective conjugated positions with the fundus Er.

As the optical path branching part 34 in the example, a flip-up mirror is used. However, any optical element other than the flip-up mirror may be adopted as the optical path branching part 34. For example, a dichroic mirror that separates visible light and infrared light by wavelength may be used. The optical path branching part (the flip-up mirror) 34 is driven by the drive mechanism 39. Specifically, the drive mechanism 39 is activated to insert the flip-up mirror in the optical path during observation of the fundus Er or remove the flip-up mirror from the optical path during photographing of fundus Er.

The observation-photographing optical system 30 includes, between the examinee's eye E and the optical path branching part 34, the objective lens 25, the perforated mirror 22, a photographing diaphragm 31, a focusing lens 32, and an image forming lens 33. An optical path formed from the objective lens 25 to the optical path branching part 34 is a common optical path of the fundus reflection light formed by the infrared light and the fundus reflection light formed by the visible light. The focusing lens 32 is driven by a movement mechanism 49 to move along an optical axis L1.

On an optical path in a reflection direction of the optical path branching part 34, there are disposed a dichroic mirror 37, a relay lens 36, and the imaging device 38 (for observation) in this order. The dichroic mirror 37 in the present embodiment has the property of reflecting infrared light (observation light) and transmitting visible light (stimulation light). The fundus photographing optical system in the present embodiment shares the optical path from the objective lens 25 to the image forming lens 33 with the fundus observation optical system.

With the above-mentioned structure, the imaging device 38 captures a front image of the fundus Er illuminated by the infrared light as a moving image or a still image. The imaging device 35 captures an image of the fundus Er illuminated by the visible light as a still image. The front image of the fundus captured by each of the imaging devices 35 and 38 is displayed on the monitor 8.

The present embodiment is one example and thus the fundus Er have only to be captured. For example, a laser beam may be scanned over the fundus Er to obtain a fundus image of the examinee's eye E.

### <Examination Optical System>

The examination optical system 70 (the "light projecting optical system" in the example) is configured to irradiate the stimulation light to an examination area on the fundus Er. As shown in FIG. 5, the examination optical system 70 shares a part of the optical path with the observation-photographing optical system 30. On an opposite side of the optical path branching part 34 (the flip-up mirror) and a transmission side of the dichroic mirror 37, an independent optical path L2 of the examination optical system 70 is formed. In this independent optical path L2 of the examination optical system 70, a display device 72 is placed.

The display device 72 is placed in a conjugated position with the fundus Er. The display device 72 can display a stimulation target. The stimulation target displayed on the display device 72 is used as a stimulus to the retina of the examinee's eye E. In the present example, background light is emitted from the display device 72. In the display device 72, a light source of the background light and a light source of stimulation light may be communalized.

The display device 72 may be configured to change the gray-scale of each of the stimulation target and the fixation target to be projected onto the fundus Er (that is, the luminance of the stimulation light). The display device 72 may further be configured to arbitrarily change the size and the shape of each of the stimulation target and the fixation target. The control unit (one example of control means) 80 can change the display position of a target to be displayed on the display device 72 to thereby change the projection position of the target on the fundus Er. In the present example, the display device 72 is a projector and includes a light source that emits white light, an LCD (a liquid crystal display), and others.

In the display device 72, a white LED is preferable as the light source that emits white light. The white LED can emit the light having a good color rendering property in a wavelength band defined from the intensity peak of blue cone cells to the intensity peak of red cone cells. The white LED is good in controllability of light quantity, less in aged deterioration, long in service life, and good in availability.

The structure for generating the stimulation target is not limited to the display device 72. For example, it may be arranged to scan a laser beam over the fundus Er to form various targets on the fundus Er (on the retina). In this case, the light projecting optical system 70 includes a laser source and an optical scanner instead of the display device 72.

In the present example, the display device 72 can display a fixation target. The fixation target may be displayed together with the examination target(s). The fixation target displayed by the display device 72 is used to guide the visual line of the examinee's eye E. As described above, the distribution of the rod cells is less in the central fovea and more in the peripheral area. Accordingly, in the Scotopic test, if a mere light spot is projected as a fixation target, the examinee's eye E may not visually recognize the fixation target when the examinee's eye E turns to a predetermined direction. In the Scotopic test, therefore, for example, the display device 72 displays a ring-shaped fixation target to form and project a fixation target of ring-shaped light onto the surrounding area of the central fovea. The ring-shaped light may also be projected to a nearly fixed position while being slightly moved. At that time, the ring-shaped light may be enlarged, reduced, or lenticulated. Since the rod cells are highly responsive to a moving object, fixation can be induced.

Furthermore, the examination optical system 70 further includes a filter 75. The filter 75 is inserted in and removed from an independent optical path L2 by the drive part 76. For the Photopic test, the filter 75 is removed from the optical path L2. For the Scotopic test, the filter 75 is inserted in the optical path L2. The filter 75 switches the wavelength band of stimulation light to the first wavelength band (the wavelength band being defined on a longer wavelength side than the sensitivity peak of blue cone cells and on a shorter wavelength side than the sensitivity peak of green cone cells, and including at least the sensitivity peak of rod cells) and reduces the luminance of the stimulation light and the luminance of the background light.

One of the two surfaces of the filter 75 intersecting the optical axis L2 is provided with a bandpass filter 75a and the other is provided with an ND filter 75b. The filter 75 is designed as an optical member integrally including both the bandpass filter 75a and the ND filter 75b. In the present example, the bandpass filter 75a and the ND filter 75b are each formed as a coated layer formed on opposite surfaces of a glass material by coating.

The ND filter 75b is one kind of neutral density filters and reduces the luminance at a uniform ratio in each wavelength, as shown in FIG. 6. This ND filter 75 can reduce the luminance of the light allowed to pass through the bandpass filter 75a to a desired luminance. In the present example, the ND filter 75b may have a transmittance ratio of about 10% to 20%. In the present example, the luminance of the stimulation light to be irradiated to the examinee's eye E in the Scotopic test is limited to a range of 0.007 asb or lower. This luminance condition may be satisfied by combination of insertion of the filter 75 and luminance control in the display device 72 or by only insertion of the filter 75.

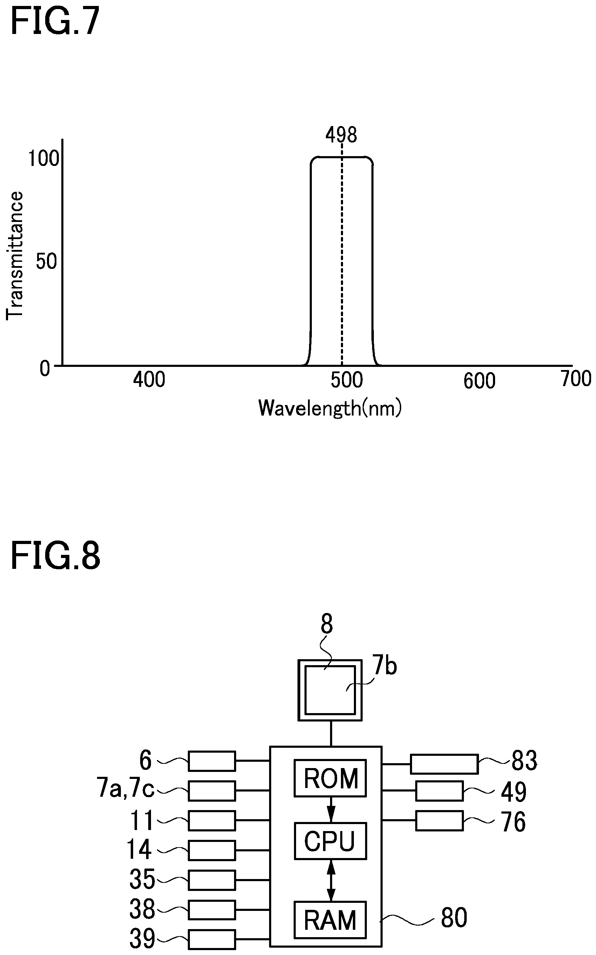

The bandpass filter 75a has such a wavelength transmission property as shown in FIG. 7. Specifically, this property exhibits a peak at 498 nm coincident with the sensitivity peak of rod cells and has a full width at half maximum of 40 nm. However, the property is not limited thereto. For example, in the case where the peak of a transmission region of the bandpass filter 75a coincides with the sensitivity peak of rod cells, the full width at half maximum may fall within a range of 10 nm to 70 nm and of course may lie within a range of 10 nm or less. Further, the peak of the bandpass filter 75a may be slightly deviated from the sensitivity peak of the rod cells. The transmission region of the bandpass filter 75a may also be biased toward either a long wavelength side or a short wavelength side. Further, the wavelength band allowed to pass through the bandpass filter 75a (that is, the first wavelength band) may be appropriately set, by also taking account of the luminance of the stimulation light, in a range that a stimulation target during the Scotopic test is prevented from being visually recognized by influence of light stimulation to the cone cells.

The light from the white LED has wavelengths appropriately distributed even on a blue side. In the case where the white LED is adopted as the light source of stimulation light, therefore, an apparatus using the short pass filter as in Patent Document 1 is conceived as causing the blue cone cells to be easily stimulated by the stimulation light even during the Scotopic test. In contrast, in the present example, the bandpass filter 75a appropriately cuts the wavelength band to which the blue cone cells are sensitive. The apparatus in the present example can execute the Scotopic test well even adopting the white LED.

### <Control System>

The control system of the apparatus 1 will be described below with reference to FIG. 8. The apparatus 1 includes the control unit 80 as a main control system. The control unit 80 is configured to control operations of the entire apparatus 1. In the example, the control unit 80 includes a CPU (a processor), a RAM, a ROM, and others. The control unit 80 is connected to a memory 83. This memory 83 stores in advance various programs, a plurality of test patterns of different types, initial values, and others. The memory 83 can store various information such as photographed images.

Furthermore, the control unit 80 is connected to the drive part 6, the illumination light source 11, the photographing light source 14, the imaging device 35, the imaging device 38, the drive part 76, the movement mechanism 49, the display device 72, the UI 7(the joystick 7a, the controller 7b, the response button 7c), the monitor 8, and others.

### <Explanation of Operations>

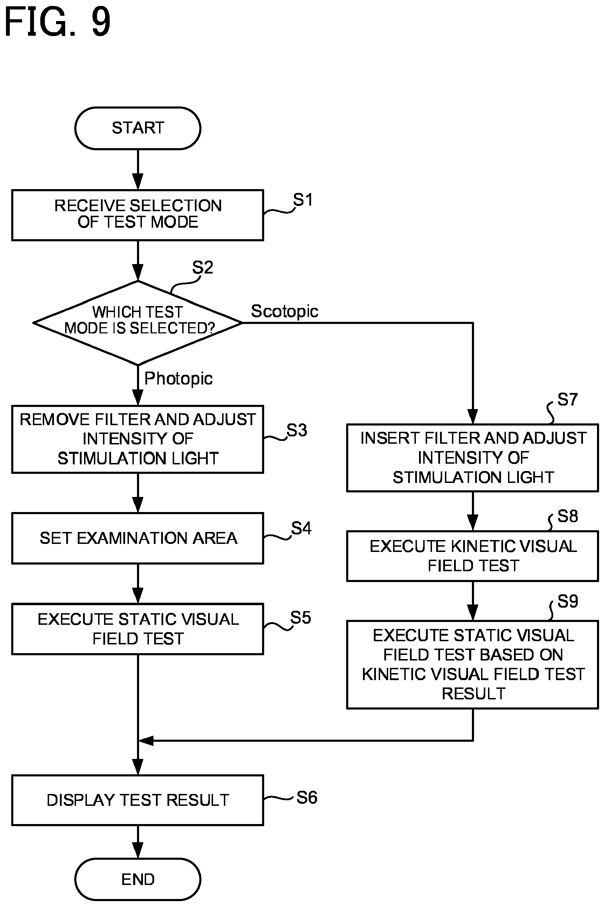

The operations of the apparatus 1 to be used for the visual function test will be described below with reference to a flowchart in FIG. 9.

Prior to the test, the apparatus 1 receives an order to select the test mode input by an examiner (S1). At that time, the test mode may be selected between at least the Photopic test mode and the Scotopic test mode. By operation on the controller 7b and others, any one of the test modes is selected.

### (Common Operations in Each Mode)

In each of the test modes, the illumination light source 11 continuously emits infrared light to the fundus Er. An observation image based on the fundus reflection light formed by the infrared light is obtained based on a signal from the imaging device 38 of the observation-photographing optical system 30. Based on this observation image, the position of the optical system is appropriately aligned with the examinee's eye E. In this appropriate alignment state, the test is started. In the present example, further, during the test, the observation image formed by the infrared light is continuously obtained. During the test, tracking is performed based on the observation image. Specifically, a display position of the stimulation target in the display device 72 may be controlled based on the observation image to present the stimulation target to a desired position on the observation image.

### (Photopic Test Mode)

When receiving an order to select the Photopic test mode as the test mode to be performed (S2: Photopic), the control unit 80 sets the irradiation condition of stimulation light to a condition for the Photopic test (S3). Herein, the control unit 80 removes the filter 75 from the optical path L2 and further adjusts the luminance of the stimulation light to be emitted from the display device 72. Consequently, the wavelength band of the stimulation light is set to the second wavelength band. In the Photopic test, adjusting the luminance of the stimulation light may be performed by control of the light source included in the display device 72. During this test, the control unit 80 also controls the display device 72 to present a fixation target.

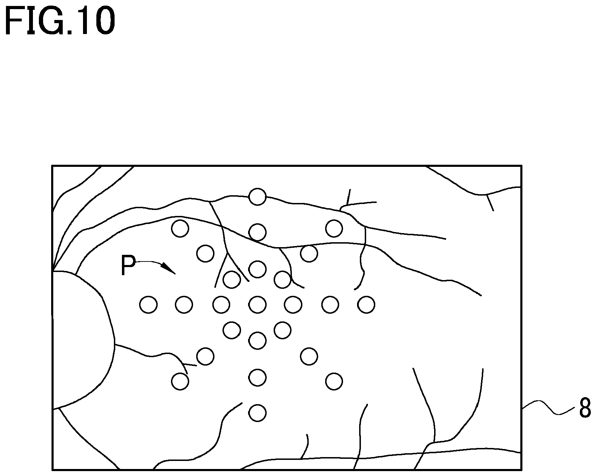

In the present example, the static visual field test is executed in the Photopic test mode. Prior to this test, an area to be examined (an examination area) is specified (S4). The control unit 80 firstly obtains a fundus image through the photographing-observation optical system 30 and displays the image on the monitor 8. The examiner operates the controller 7b to specify the examination area. This examination area is a retinal portion to be optically stimulated to examine its visual function. For instance, a test pattern P which is a placement pattern of the examination area is selected and then the position of the test pattern P with respect to the fundus is set through the fundus image on the monitor 8 (see FIG. 10).

Successively, the examiner operates the controller 7b to start the test (S5). The control unit 80 displays, in the display device 72, one of a plurality of test targets included in the test pattern P at a predetermined level of brightness. While staring at the fixation target presented at the center of the visual field, the examinee is asked to press the response button 7c as soon as he/she perceives the test target on the periphery of the visual field. When the control unit 80 detects that the response button 7c has been pressed or upon time-out, the control unit 80 causes a next test target to be displayed. The control unit 80 then stores the examination area (the display position of the test target) and the stimulation luminance (the bright ness of the examination target) in association with the presence/absence of a response by the examinee in the memory 83. The control unit 80 repeatedly changes the display position of the test target to another and changes the brightness of each displayed test target. After the control unit 80 obtains a threshold value corresponding to each test target of the test pattern P, that is, the boundary between the brightness at which a test target is visible and the brightness at which the same test target is invisible, the control unit 80 terminates the visual function test. The obtained threshold values are stored in the memory 83 in association with the display positions of the test target.

The control unit 80 then obtains a color fundus image and displays on the monitor 8 a composite image formed by merging the color fundus image and a threshold map as a visual function test result (S6). To be concrete, the control unit 80 performs a registration processing (enlargement, reduction, rotation, etc.) on a photographed image to make each portion of a reference image and each portion of the photographed image nearly coincide with each other and further superimposes the information on the threshold value onto the examination area of the fundus image corresponding to each test target of the test pattern P.

### (Scotopic Test Mode)

In the Scotopic test mode, an examinee is kept waiting in a dark room for about several tens of minutes prior to the test. This is to enhance the sensitivity of the rod cells of the examinee in preparation for the Scotopic test.

Herein, the control unit 80 sequentially executes the kinetic test and the static test as the Scotopic test. The kinetic test is performed first and then the static test is performed based on a test result of the kinetic test.

When the Scotopic test mode is selected (S2: Scotopic), the control unit 80 sets the irradiation condition of stimulation light to a condition for the Scotopic test (S7). Herein, the control unit 80 inserts the filter 75 into the optical path L2 and further adjusts the luminance of the stimulation light to be emitted from the display device 72. Consequently, the wavelength band of the stimulation light is switched to the first wavelength band and the luminance of the background light is limited to 0.007 asb or less (the luminance corresponding to the dark adaptation in the present example). The control unit 80 controls the display device 72 to present a ring-shaped fixation target during the test.

The kinetic visual field test is subsequently executed (S8). In this kinetic visual field test, the control unit 80 controls the display device 72 to move the display position of the stimulation target on the display device 72 from the periphery to the center. Accordingly, the position of the stimulation target to be presented is moved from the periphery to the center of the fundus Er. The examinee operates the response button 7c at the time when he/she visually recognizes the stimulation target. The control unit 80 stores data indicating the position of the stimulation target (e.g., the position relative to the central fovea) determined when the response button 7c is pressed, as response position data in the memory 83. The control unit 80 changes the luminance of the stimulation target and moves the presenting position of the stimulation target along the same path (i.e., from the periphery to the center of the fundus Er), and repeats the test to obtain the response position data in that path at each luminance of the stimulation target. The response position data may include data on positions on the fundus image. The same operations are performed in different meridian directions. The resultant response position data is equivalent to distribution data of the luminosity of the rod cells. Further, the control unit 80 produces isopters based on the response position data. The isopters are merged with the fundus image obtained by the observation-photographing optical system 30 and displayed on the monitor 8.

In the present example, the static test is then executed (S9). The operations in this test are almost the same as those in the Photopic test and therefore only the operations specific to the Scotopic test will be explained below. The luminosity distribution obtained in the kinetic visual field test includes an error(s) due to time lag from when the examinee visually recognizes the stimulation target and to when the examinee operates, or presses, the response button 7c. In the static test, therefore, an initial value of the luminance of the stimulation light in each examination area is set to be equal to or larger by a predetermined amount than the luminance corresponding to the luminosity obtained in the kinetic test. The rod cells are distributed more in the peripheral area and thus a wide range of the fundus needs to be subjected to the test. However, the initial value of the luminance of the stimulation light in the static test is set based on the distribution data of the luminosity obtained in the kinetic test, so that a brief test can be realized.

The control unit 80 subsequently obtains a color fundus image and displays a composite image formed by merging the color fundus image and the threshold map, as a visual function test result (S6).

The present disclosure is described along the foregoing embodiment, but it is not limited to the above embodiment. The present disclosure may be embodied in other specific forms without departing from the essential characteristics thereof.

For instance, in the foregoing embodiment, the visual function test apparatus is the subjective test apparatus for obtaining a subjective reaction of an examinee with respect to stimulation light. Alternatively, the visual function test apparatus may be an objective test apparatus. For instance, the objective test apparatus may be configured to automatically obtain a physiological reaction of an examinee to the irradiation of stimulation light. For instance, a reaction of the examinee may be obtained as a change in the fundus image between before and after irradiation of the stimulation light or may be acquired as an electric physiological reaction obtained by for example an electroretinograph (ERG) test device.

In the foregoing embodiment, the perimeter which is one example of the visual function test apparatus is exemplified as a microperimeter, but is not limited thereto and may be exemplified as another perimeter such as a Humphrey perimeter and a Goldmann perimeter.

The example shows the Scotopic test in which the kinetic test is performed and then continuously the static test is executed based on the test result of the kinetic test. However, the present disclosure is not limited thereto. For instance, the static test based on the test result of the kinetic test may be performed on a different day from the day when the kinetic test is conducted. As the Scotopic test, only either the kinetic test or the static test may be performed.

## Claims

1. A visual function test apparatus comprising:
a light projecting optical system (70) configured to irradiate stimulation light and background light to an examination area of a fundus (Er) of an eye (E) of an examinee;
reaction obtaining means (7c) configured to obtain a reaction of the examinee to the stimulation light; and
control means (80) configured to control at least an irradiation position of the stimulation light to be irradiated from the light projecting optical system to the examination area,
wherein the light projecting optical system (70) is configured to irradiate the background light at a luminance corresponding to dark adaptation and irradiate the stimulation light and the background light in a first wavelength band having a lower limit on a longer wavelength side than a sensitivity peak of blue cone cells and an upper limit on a shorter wavelength side than a sensitivity peak of green cone cells, the first wavelength band being a wavelength band including at least a sensitivity peak of rod cells.

2. The visual function test apparatus according to claim 1, wherein the light projecting optical system (70) includes a bandpass filter (75a) configured to transmit the first wavelength band and further shield light on a longer wavelength side and a shorter wavelength side than the first wavelength band.

3. The visual function test apparatus according to claim 1 or 2, wherein the light projecting optical system (70) includes a neutral density filter (75b) having a property of darkening light in the first wavelength band.

4. The visual function test apparatus according to claim 1, further including irradiation condition switching means configured to switch an irradiation condition for the stimulation light and the background light in the light projecting optical system (70) between
a first irradiation condition for irradiating the stimulation light and the background light in the first wavelength band to perform a visual function test under a dark adaptation condition, and
a second irradiation condition for irradiating the stimulation light and the background light in a second wavelength band different from the first wavelength band and to which various blue, green, and red cone cells are sensitive to perform a visual function test under a light adaptation condition.

5. The visual function test apparatus according to claim 4, wherein the irradiation condition switching means includes at least a bandpass filter (75a) configured to transmit the first wavelength band and further shield light on a longer wavelength side and a shorter wavelength side than the first wavelength band and an insertion/removal mechanism (76) for inserting and removing the bandpass filter with respect to an optical path (L2) of the light projecting optical system (70).

6. The visual function test apparatus according to claim 4, wherein the irradiation condition switching means further includes a neutral density filter (75b) having a property of darkening light in the first wavelength band and an insertion/removal mechanism (76) for inserting and removing the neutral density filter with respect to an optical path (L2) of the light projecting optical system (70).

7. The visual function test apparatus according to claim 4, wherein
the irradiation condition switching means (75) includes a filter (75) integrally including a bandpass filter (75a) configured to transmit the first wavelength band and further shield light on a longer wavelength side and a shorter wavelength side than the first wavelength band and a neutral density filter (75b) having a property of darkening the light in the first wavelength band, and
when the filter (75) is inserted or removed with respect to an optical path (L2) of the light projecting optical system (70), the bandpass filter and the neutral density filter are inserted or removed together.

8. The visual function test apparatus according to claim 7, wherein the neutral density filter (75b) and the bandpass filter (75a) are formed in different layers from each other.

9. The visual function test apparatus according to any one of claims 4 to 8, wherein
the irradiation condition switching means further includes a light source (72) of the stimulation light and the background light, and the control means (80), and
at least the light source is controlled to switch the irradiation condition between the first irradiation condition and the second irradiation condition.

10. The visual function test apparatus according to claim 6 or 7, wherein
the irradiation condition switching means further includes a light source (72) of the stimulation light and the background light, and the control means (80), and
an amount of light to be emitted from the light source is controlled in combination with insertion and removal of the neutral density filter to set the background light to a luminance corresponding to dark adaptation under the first irradiation condition.

11. The visual function test apparatus according to any one of claims 1 to 10, further including an imaging optical system (30) configured to irradiate infrared light from an illumination light source (11) to the examinee's eye (E) and receive reflection light formed by the infrared light from the examinee's eye to obtain a front image of the examinee's eye, and
wherein the infrared light to be emitted to the examinee's eye by the imaging optical system (30) is light having a wavelength on a longer wavelength side than a wavelength band to which red cone cells are substantially sensitive.

12. The visual function test apparatus according to any one of claims 3, 5, 7 and 8, further including optical-path combining means (37) configured to combine an optical path (L1) of the imaging optical system (30) and an optical path (L2) of the light projecting optical system (70), and
wherein the bandpass filter (75a) is placed in an independent optical path (L2) of the light projecting optical system (70).

13. A visual function test method using a visual function test apparatus comprising:
a light projecting optical system (70) configured to irradiate stimulation light and background light to an examination area of a fundus (Er) of an eye (E) of an examinee;
reaction obtaining means (7c) configured to obtain a reaction of the examinee to the stimulation light; and
control means (80) configured to control at least an irradiation position of the stimulation light to be irradiated from the light projecting optical system to the examination area,
wherein the light projecting optical system (70) is configured to irradiate the background light at a luminance corresponding to dark adaptation and irradiate the stimulation light and the background light in a first wavelength band having a lower limit on a longer wavelength side than a sensitivity peak of blue cone cells and an upper limit on a shorter wavelength side than a sensitivity peak of green cone cells, the first wavelength band being a wavelength band including at least a sensitivity peak of rod cells.
